(19) Europäisches Patentamt
European Patent Office
Office européen des brevets

(11) Veröffentlichungsnummer: **0 300 358**
**B1**

(12) **EUROPÄISCHE PATENTSCHRIFT**

(45) Veröffentlichungstag der Patentschrift: **19.12.90**

(51) Int. Cl.⁵: **C07D 301/26**, C07D 303/16

(21) Anmeldenummer: **88111284.1**

(22) Anmeldetag: **14.07.88**

(54) **Verfahren zur Herstellung von Perfluoralkyl enthaltenden Epoxiden und neue Perfluoralkyl enthaltende Epoxide.**

(30) Priorität: **22.07.87 DE 3724198**

(43) Veröffentlichungstag der Anmeldung:
**25.01.89 Patentblatt 89/4**

(45) Bekanntmachung des Hinweises auf die Patenterteilung:
**19.12.90 Patentblatt 90/51**

(84) Benannte Vertragsstaaten:
**AT BE CH DE FR GB IT LI NL**

(56) Entgegenhaltungen:
**FR-A- 2 529 890**

**CHEMICAL ABSTRACTS, Band 102, Nr. 3, 21 Januar 1985, Columbus, Ohio,USA BRYCE; M.R.; CHAMBERS, R.D.; KIRK, J.R. "Reactions involving fluoride ion. Part 30. Preparation and reactions of epoxides derived from perfluoroalkyl substituted alkenes." Seite 670, Spalte 1, Zusammenfassung Nr. 24 378e 000**

(73) Patentinhaber: **HOECHST AKTIENGESELLSCHAFT, Postfach 80 03 20, D-6230 Frankfurt am Main 80(DE)**

(72) Erfinder: **Wehowsky, Frank, Dr., Talhauser Strasse 27, D-8269 Burgkirchen(DE)**

**EP 0 300 358 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung von Perfluoralkyl enthaltenden Epoxiden. Die Erfindung betrifft ferner neue Perfluoralkyl enthaltende Epoxide.

Aus der US-Patentschrift 4 709 074 sind Addukte bekannt, die aus einem Perfluoralkyl enthaltenden Alkohol (Fluoralkohol) und Epichlorhydrin aufgebaut sind. Sie werden als Ausgangsverbindungen zur Herstellung von fluorhaltigen Urethanen eingesetzt.

Aus der US-Patentschrift 4 267 302 sind Verbindungen der folgenden Formel bekannt,

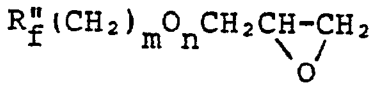

in der $R_f''$ ein Perfluoralkylrest mit 1 bis 18 C-Atomen, m 0 oder eine ganze Zahl von 1 bis 3 und n 0 oder 1 ist. Bezüglich der Herstellung dieser Perfluoralkyl enthaltenden Epoxide wird lediglich auf zwei Reaktionsgleichungen verwiesen, von denen die hier in Betracht zu ziehende nachstehend wiedergegeben wird:

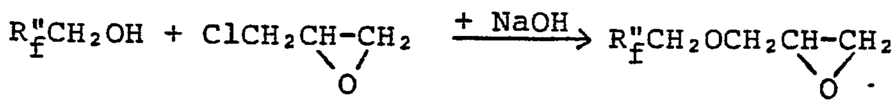


Bei der nach dieser Gleichung ausgeführten Umsetzung entsteht das angegebene Epoxid, wie ein Versuch gezeigt hat, in nur geringer Ausbeute.

Die Aufgabe der Erfindung besteht demnach darin, ein verbessertes Verfahren zur Herstellung der aus der genannten US-PS 4 267 302 bekannten Perfluoralkyl enthaltenden Epoxide und weiterer derartiger Verbindungen bereitzustellen.

Das erfindungsgemäße Verfahren zur Herstellung von Perfluoralkyl enthaltenden Epoxiden der folgenden Formel 1

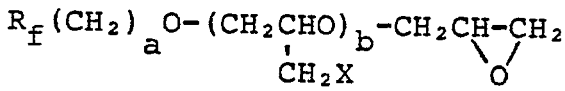

(1) ,

worin bedeuten

$R_f$ einen Perfluoralkylrest mit 4 bis 20 C-Atomen, vorzugsweise 6 bis 16 C-Atomen, einen ω-H-Perfluoralkylrest mit 4 bis 20 C-Atomen, vorzugsweise 6 bis 16 C-Atomen, oder einen Rest der Formel $R_f'SO_2NR_3$ oder der Formel $R_f'CH_2CH_2SO_2NR_3$, worin $R_f'$ ein Perfluoralkylrest mit 4 bis 20 C-Atomen, vorzugsweise 6 bis 16 C-Atomen, und $R_3$ = H oder ein $C_{1-4}$-Alkyl ist; von diesen Bedeutungen von $R_f$ ist der Perfluoralkylrest mit 4 bis 20 C-Atomen, vorzugsweise 6 bis 16 C-Atomen, bevorzugt,

X Br, Cl oder J, vorzugsweise Br oder Cl,

a eine ganze Zahl von 1 bis 4, vorzugsweise 2, und

b eine Zahl von 0 bis 20, vorzugsweise 0 bis 10,

ist dadurch gekennzeichnet, daß ein Fluoralkohol-Epihalogenhydrin-Addukt der folgenden Formel 2

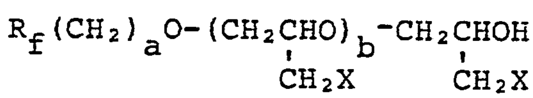

(2) ,

worin $R_f$, X, a und b die genannten Bedeutungen haben,

zur Abspaltung von Halogenwasserstoff unter Cyclisierung der (endständigen) Halogenhydringruppe des Adduktes mit Alkalimetall- oder Erdalkalimetallhydroxiden in Form einer wäßrigen Lösung, in Gegenwart von Phasentransferkatalysatoren und bei einem pH-Wert von 9 bis 12, vorzugsweise 10 bis 11, umgesetzt und die gebildete, angestrebte Epoxidverbindung aus der Reaktionsmischung isoliert wird. Der Perfluoralkylrest $R_f$ in den Formeln 1 und 2 kann geradkettig oder verzweigt sein, er ist vorzugsweise geradkettig. $R_f$ stellt in der Regel ein Gemisch von Perfluoralkylresten mit der oben angegebenen Anzahl von C-Atomen dar.

Durch die Kombination der drei erfindungsgemäßen Maßnahmen, das sind der Einsatz von Alkalimetall- oder Erdalkalimetallhydroxiden in Form einer wäßrigen Lösung, der Einsatz von Phasentransferkatalysatoren und die Aufrechterhaltung eines pH-Wertes von 9 bis 12, vorzugsweise 10 bis 11, während der Umsetzung, wird eine beschleunigte und vollständige, unter Cyclisierung verlaufende Abspaltung von Halogenwasserstoff von der (endständigen) Halogenhydringruppe des in Rede stehenden Adduktes erreicht, mit dem weiteren großen Vorteil, daß unerwünschte Nebenprodukte, wenn überhaupt, nur in

geringer Menge gebildet werden.

Die als Ausgangsverbindungen einzusetzenden Fluoralkohol-Epihalogenhydrin-Addukte sind in der eingangs genannten US-Patentschrift 4 709 074 ausführlich beschrieben. Sie stellen bei Raumtemperatur je nach Länge des $R_f$-Restes und je nach Anzahl der im Molekül vorhandenen Chlormethyloxethylen-Einheiten, flüssige bis wachsartig feste, farblos oder gelb bis braun gefärbte Produkte dar. Sie werden hergestellt durch Umsetzung eines Fluoralkohols der Formel $R_f(CH_2)_a$-OH, worin $R_f$ und a die genannten Bedeutungen haben, mit Epihalogenhydrin bei einer Temperatur von 30 bis 150 °C, vorzugsweise 40 bis 90 °C, in Gegenwart von Lewis-Säure als Katalysator, wobei der Alkohol und das Epihalogenhydrin im Molverhältnis von 1 zu b + 1 eingesetzt werden (b hat die in Formel 2 genannte Bedeutung). Die Umsetzung wird, wie in der genannten US-Patentschrift ausführlich beschrieben ist, in Substanz oder unter Verwendung eines organischen Lösungsmittels durchgeführt. Die Umsetzung verläuft quantitativ. Eingesetzte Lewis-Säure kann durch Waschen mit alkalischen Mitteln und eingesetztes Lösungsmittel durch Destillation entfernt werden. Geeignete Lewis-Säuren sind $BF_3$, Bortrifluoriddiethyletherat, $SnCl_4$, $SbCl_5$, $TiCl_4$, $FeCl_3$, $PF_5$ und/oder Dibutylzinndilaurat, wobei Bortrifluoriddiethyletherat und Dibutylzinndilaurat bevorzugt sind. Geeignete Lösungsmittel sind halogenierte Kohlenwasserstoffe, wie Tetrachlorkohlenstoff (Siedepunkt 77 °C), Dichlorethan (Siedepunkt 84 °C) und Trifluortrichlorethan (Siedepunkt 48 °C), Ketone,wie Aceton (56 °C), Methylethylketon (80 °C) und Diethylketon (101 °C), Ether,wie Diisopropylether (68 °C), Tetrahydrofuran (66 °C) und Dibutylether (142 °C),und Ester,wie Ethylacetat (77 °C) und Butylacetat (123 °C).

Die Fluoralkohol-Epihalogenhydrin-Addukte der Formel 2 werden gemäß Erfindung mit Alkalimetall- oder Erdalkalimetallhydroxid umgesetzt. Von den beiden Metallhydroxiden sind die Alkalimetallhydroxide bevorzugt. Von diesen werden aus Zweckmäßigkeitsgründen vorzugsweise Natriumhydroxid und Kaliumhydroxid eingesetzt. Die Menge an Metallhydroxid richtet sich nach der Menge an abzuspaltendem und zu bindendem Halogenwasserstoff (HX). Das Metallhydroxid wird also in einer stöchiometrischen Menge, bezogen auf die Menge an HX,in der (endständigen) Halogenhydringruppe des Fluoralkohol-Epihalogenhydrin-Adduktes eingesetzt, das heißt, etwa ein Mol Alkalimetallhydroxid oder ein halbes Mol Erdalkalimetallhydroxid pro Mol Fluoralkohol-Epihalogenhydrin-Addukt. Das Metallhydroxid wird in Form einer wäßrigen Lösung eingesetzt. Die Konzentration dieser Lösungen kann in weiten Grenzen variieren, entscheidend ist lediglich, daß die eingesetzte Lösung die für die Abspaltung von HX erforderliche Menge an Metallhydroxid enthält. Im allgemeinen werden Lösungen verwendet, die 1 bis 40 Gew.-% Metallhydroxid enthalten, vorzugsweise 4 bis 20 Gew.-%, Gewichtsprozente bezogen auf die wäßrige Lösung, das ist die Gewichtssumme aus Wasser und Metallhydroxid.

Nach dem zweiten Merkmal des erfindungsgemäßen Verfahrens wird die in Rede stehende Reaktion in Gegenwart von Phasentransferkatalysatoren durchgeführt. Mit Hilfe dieser Katalysatoren wird erreicht, daß ein Austausch stattfindet zwischen der organischen Phase, das ist das Fluoralkohol-Epihalogenhydrin-Addukt,und der wäßrigen Phase, das ist die wäßrige Metallhydroxidlösung, einschließlich jener Menge an Wasser, die gegebenenfalls eingesetzt worden ist, um das Fluoralkohol-Epihalogenhydrin-Addukt in eine Dispersion zu bringen (diese Menge beträgt etwa 0,5 bis 2 Gewichtsteile Wasser pro Gewichtsteil Addukt). Aus der großen Zahl der bekannten Phasentransferkatalysatoren haben sich die folgenden Vertreter als besonders geeignet erwiesen: Tetraalkylammonium- und Tetraalkylphosphoniumverbindungen, wobei von den vier Alkylresten ein oder zwei Alkylreste jeweils 4 bis 18 C-Atome enthalten und die übrigen Alkylreste jeweils 1 bis 4 C-Atome enthalten, wie Tetrabutylammoniumbromid, Dioctyldimethylammoniumchlorid, Dicetyldimethylammoniumchlorid, Dodecyltrimethylammoniumhydrogensulfat und die analogen Phosphoniumverbindungen; Polyglykolether der Formel $RO(CH_2CH_2O))_xR'$, worin R vorzugsweise $C_{1-3}$-Alkyl, x eine Zahl von vorzugsweise 3 bis 15 und R' = H (das sind Polyethylenglykolmonoalkylether) oder ebenfalls vorzugsweise $C_{1-3}$-Alkyl ist (das sind Polyethylenglykoldialkylether); Kronenether, wie [15]Krone-5, [18]Krone-6, Dibenzo-[18]krone-6 und Dicyclohexyl-[18]krone-6; und Kryptanden, wie Kryptand220, Kryptand221 und Kryptand222. Der Phasentransferkatalysator wird in einer Menge von etwa 0,05 bis 5 Gew.-% eingesetzt, vorzugsweise in einer Menge von 0,1 bis 3 Gew.-%, Gewichtsprozente bezogen auf das Gewicht des eingesetzten Fluoralkohol-Epihalogenhydrin-Adduktes.

Nach dem dritten Merkmal des erfindungsgemäßen Verfahrens wird die Umsetzung des Fluoralkohol-Epihalogenhydrin-Adduktes mit der wäßrigen Lösung von Alkalimetall- oder Erdalkalimetallhydroxid in Gegenwart von Phasentransferkatalysatoren bei einem pH-Wert von 9 bis 12, vorzugsweise 10 bis 11, durchgeführt, das heißt, in der wäßrigen Phase der Reaktionsmischung soll während der Umsetzung der angegebene pH-Wert vorliegen. Bei einem pH-Wert von kleiner als 9 verläuft die Umsetzung nur noch sehr langsam, während bei einem pH-Wert von größer als 12 neben der angestrebten Cyclisierung zum Epoxid auch schon Polymerisation des gebildeten Epoxids eintritt. Die Kontrolle des pH-Wertes während der Umsetzung kann durch laufende Messung des pH-Wertes oder mit Hilfe eines in der Reaktionsmischung vorhandenen geeigneten Indikators erfolgen.

Das erfindungsgemäße Verfahren wird vorzugsweise wie folgt durchgeführt: Das Fluoralkohol-Epihalogenhydrin-Addukt, dispergiert in Wasser zu einer Dispersion (dazu werden etwa 0,5 bis 2 Gewichtsteile pro Gewichtsteil Addukt eingesetzt), und der Phasentransferkatalysator werden in einem Reaktionsgefäß vorgelegt. Die Mischung wird auf eine Temperatur von 30 bis 100 °C, vorzugsweise 40 bis 80

°C erhitzt (Reaktionstemperatur). Zur Mischung wird nun unter Rühren die zur Abspaltung von Halogenwasserstoff aus der Halogenhydringruppe des Adduktes stöchiometrisch erforderliche Menge an Metallhydroxid in Form einer wäßrigen Lösung zugegeben (portionsweise oder kontinuierlich) und dabei ein pH-Wert (in der wäßrigen Phase) von 9 bis 12, vorzugsweise 10 bis 11, eingehalten.

Nach Beendigung der Zugabe des Metallhydroxids wird die Reaktionsmischung zur Vervollständigung der Umsetzung bei der angegebenen Reaktionstemperatur noch einige Zeit gehalten (die Umsetzung kann beispielsweise durch Bestimmung der Epoxidzahl in der Reaktionsmischung verfolgt werden). Nach Beendigung der Umsetzung wird die organische Phase, das ist die angestrebte Epoxidverbindung gemäß Formel 1, von der wäßrigen Phase abgetrennt (die wäßrige Phase enthält das bei der Abspaltung von Halogenwasserstoff gebildete Metallhalogenid). Die abgetrennte Epoxidverbindung kann zur Reinigung mit Wasser gewaschen werden; dabei werden eventuelle Verunreinigungen, wie Metallhydroxid, Metallhalogenid und/oder Phasentransferkatalysator entfernt.

Das erfindungsgemäße Verfahren liefert die angestrebte Epoxidverbindung in hoher Ausbeute. Unerwünschte Nebenprodukte entstehen, wenn überhaupt, nur in sehr geringer Menge. Die erhaltenen Epoxidverbindungen stellen bei Raumtemperatur, je nach Länge des $R_f$-Restes und der Anzahl der gegebenenfalls vorhandenen Halogenmethyloxethylen-Einheiten, flüssige bis feste (wachsartig), farblose bis gelb oder braun gefärbte Produkte dar.

Von den Perfluoralkyl enthaltenden Epoxiden der oben angegebenen Formel 1 sind jene mit b = 0 aus der eingangs genannten US-Patentschrift 4 267 302 bekannt, während jene mit b größer als Null neu sind.

Die Erfindung betrifft demnach neben dem oben beschriebenen Herstellungsverfahren auch neue Perfluoralkyl enthaltende Epoxide, gekennzeichnet durch die folgende Formel 3

$$R_f(CH_2)_aO-(CH_2\underset{\displaystyle CH_2X}{CH}O)_{b'}-CH_2\underbrace{CH-CH_2}_{\diagdown\, O\, \diagup} \qquad (3),$$

worin bedeuten

$R_f$ einen Perfluoralkylrest mit 4 bis 20 C-Atomen, vorzugsweise 6 bis 16 C-Atomen, einen ω-H-Perfluoralkylrest mit 4 bis 20 C-Atomen, vorzugsweise 6 bis 16 C-Atomen, oder einen Rest der Formel $R'_fSO_2NR_3$ oder der Formel $R'_fCH_2CH_2SO_2NR_3$, worin $R'_f$ ein Perfluoralkylrest mit 4 bis 20 C-Atomen, vorzugsweise 6 bis 16 C-Atomen, und $R_3$ = H oder ein $C_{1-4}$-Alkyl ist; von diesen Bedeutungen von $R_f$ ist der Perfluoralkylrest mit 4 bis 20 C-Atomen, vorzugsweise 6 bis 16 C-Atomen, bevorzugt,

X Br, Cl oder J, vorzugsweise Br oder Cl,

a eine ganze Zahl von 1 bis 4, vorzugsweise 2, und

b' eine Zahl von 1 bis 20, vorzugsweise 1 bis 10.

Die erfindungsgemäßen Verbindungen der Formel 3 können nach dem beschriebenen Verfahren hergestellt werden. Sie stellen bei Raumtemperatur, je nach Länge des $R_f$-Restes und je nach Anzahl der Halogenmethyloxethylen-Einheiten im Molekül, flüssige bis wachsartig feste, gelb bis braun gefärbte Produkte dar.

Die neuen fluorhaltigen Epoxide weisen überraschend gute Eigenschaften auf. Sie sind unerwartet leicht polymerisierbar, so daß sie ein vorteilhaftes Monomeres zur Herstellung von fluorhaltigen Epoxidharzen darstellen. Sie besitzen ferner eine unerwartet hohe Verträglichkeit mit nicht fluorierten Epoxiden,wie Phenylglycidylethern und Allylglycidylethern, und stellen deshalb auch ein vorteilhaftes Monomeres zur Herstellung von Copolymeren mit nicht fluorierten Epoxiden dar. Nachdem in der erfindungsgemäßen Verbindung die Anzahl der Halogenmethyloxethylen-Einheiten im Molekül innerhalb eines relativ weiten Bereiches variiert werden kann, ist es möglich, die Abstände zwischen dem Perfluoralkyl und dem polymeren Rückgrat in den genannten Epoxidharzen und Copolymeren gleichsam maßgeschneidert einzustellen. Die maßgeschneiderten Epoxidharze und Copolymeren stellen wertvolle Ausrüstungsmittel für Textilien und Leder dar, da sie auf diesen Substraten sehr gut haften und ihnen eine gute Oleophobie und Hydrophobie verleihen.

Die Erfindung wird nun an Beispielen noch näher erläutert:

## Beispiel 1

1 006,0 g (1,8 mol) Fluoralkohol-Epichlorhydrin-Addukt der Formel

$$C_6F_{13}CH_2CH_2O(CH_2\underset{\displaystyle CH_2Cl}{CH}O)_2-H$$

(dieses Addukt ist bei Raumtemperatur flüssig), 1 400 g Wasser zur Bildung einer Dispersion mit dem Addukt und 10,0 g $(C_8H_{17})_2N^+(CH_3)_2Cl^-$ als Phasentransferkatalysator (das sind 1 Gew.-%, bezogen auf Addukt) wurden in einem mit Rührer und Thermometer ausgestatteten Reaktionsgefäß vorgelegt. Die Mischung wurde unter Rühren auf 60 °C erhitzt. Bei dieser Temperatur wurden zur Mischung, wiederum unter Rühren, 84,0 g (2,1 mol) NaOH (100%ig), gelöst in 756 g Wasser, das ist eine 10%ige wäßrige Natriumhydroxidlösung, portionsweise (jeweils etwa 25 ml) dazugegeben und dabei in der Mischung ein pH-Wert von 11 eingehalten (die 2,1 mol NaOH entsprechen der stöchiometrischen Menge plus einem geringen Überschuß). Nachdem (nach 6 Stunden) die NaOH-Lösung zudosiert war, wurde die Mischung zur Nachreaktion noch weitere 4 Stunden lang bei 60 °C unter Rühren gehalten. Nach Abkühlen der Reaktionsmischung und Abtrennen der organischen Phase von der wäßrigen Phase, wurde die organische Phase dreimal mit je 1 l Wasser gewaschen und anschließend bei 60 °C und 1,3 kPa getrocknet. Das angestrebte Perfluoralkyl enthaltende Epoxid der Formel

$$C_6F_{13}CH_2CH_2OCH_2CHOCH_2CH\text{-}CH_2 \quad ,$$
$$CH_2Cl \qquad O$$

eine bei Raumtemperatur klare, gelbgefärbte Flüssigkeit, wurde in einer Ausbeute von 94,5 % der Theorie erhalten; die Epoxidzahl der Verbindung war 6,6, was einer Molmasse von 553 entspricht (die theoretische Epoxidzahl ist 6,8).

Beispiel 2

Ansatz:

200,0 g (0,21 mol) Fluoralkohol-Epichlorhydrin-Addukt der Formel $(C_8F_{17}\text{-}C_{16}F_{33})CH_2CH_2O(CH_2CHO)_5\text{-}H$
$$CH_2Cl$$
(bei Raumtemperatur fest),

250,0 g Wasser zur Bildung einer Dispersion mit dem Addukt,
1,2 g [15]krone-5 als Phasentransferkatalysator, das sind 0,6 Gew.-%, bezogen auf Addukt,
14,0 g (0,25 mol) KOH, gelöst in 340 g Wasser, das ist eine 4%ige wäßrige Kaliumhydroxidlösung.

Durchführung: Wie in Beispiel 1.
Das angestrebte Perfluoralkyl enthaltende Epoxid der Formel

$$(C_8F_{17}\text{-}C_{16}F_{33})CH_2CH_2O(CH_2CHO)_4CH_2CH\text{-}CH_2 \quad ,$$
$$CH_2Cl \qquad O$$

ein bei Raumtemperatur gelbgefärbtes, wachsartiges Produkt, wurde in einer Ausbeute von 95,0 % der Theorie erhalten; die Epoxidzahl der Verbindung war 3,7, was einer Molmasse von 986 entspricht (die theoretische Epoxidzahl ist 3,9).

Beispiel 3

Ansatz:

466,7 g (0,31 mol) Fluoralkohol-Epichlorhydrin-Addukt der Formel $C_8F_{17}CH_2CH_2O(CH_2CHO)_{11}\text{-}H$ (bei
$$CH_2Cl$$
Raumtemperatur fest),

700,0 g Wasser zur Bildung einer Dispersion mit dem Addukt,
9,4 g Triethylenglykoldimethylether als Phasentransferkatalysator, das sind 2 Gew.-%, bezogen auf das Addukt,
13,2 g (0,33 mol) NaOH, gelöst in 75 g Wasser, das ist eine 15%ige wäßrige Natriumhydroxidlösung.

Durchführung: Wie im Beispiel 1, mit der Ausnahme, daß die Umsetzung bei einer Temperatur von 70 °C durchgeführt und ein pH-Wert von 10 eingehalten wurde.
Das angestrebte Perfluoralkyl enthaltende Epoxid der Formel

$$C_8F_{17}\ CH_2CH_2O(CH_2\underset{CH_2Cl}{CH}O)_{10}CH_2\underset{\diagdown O \diagup}{CH-CH_2}\quad ,$$

ein bei Raumtemperatur gelbgefärbtes, wachsartiges Produkt, wurde in einer Ausbeute von 87,1 % der Theorie erhalten;
die Epoxidzahl der Verbindung war 2,3, was einer Molmasse von 658 entspricht (die theoretische Epoxidzahl ist 2,5).

Beispiel 4

Ansatz:

297,0 g (0,65 mol) Fluoralkohol-Epichlorhydrin-Addukt der Formel $C_6F_{13}CH_2CH_2OCH_2\underset{CH_2Cl}{CH}OH$ (bei Raumtemperatur flüssig),
460,0 g Wasser zur Bildung einer Dispersion mit dem Addukt,
3,0 g $(C_{10}H21_{21})2N^{+}(CH3)_2Cl^{-}$als Phasentransferkatalysator, das ist 1 Gew.-%, bezogen auf Addukt,
28,0 g (0,7 mol) NaOH, gelöst in 112 g Wasser, das ist eine 20%ige wäßrige Natriumhydroxidlösung.

Durchführung: Wie im Beispiel 1, mit der Ausnahme, daß nicht umgesetztes $C_6F_{13}CH_2CH_2OH$ nach dem Waschen abdestilliert wurde.
Das angestrebte Perfluoralkyl enthaltende Epoxid der Formel

$$C_6F_{13}\ CH_2CH_2OCH_2\underset{\diagdown O \diagup}{CH-CH_2}\quad ,$$

eine bei Raumtemperatur klare, farblose Flüssigkeit, wurde in einer Ausbeute von 75 % der Theorie erhalten;
die Epoxidzahl der Verbindung war 8,5, was einer Molmasse von 429 entspricht (die theoretische Epoxidzahl ist 8,7).

**Patentansprüche**

1. Verfahren zur Herstellung von Perfluoralkyl enthaltenden Epoxiden der folgenden Formel 1

$$R_f(CH_2)_aO-(CH_2\underset{CH_2X}{CH}O)_b-CH_2\underset{\diagdown O \diagup}{CH-CH_2}\qquad (1)\quad ,$$

worin bedeuten
$R_f$ einen Perfluoralkylrest mit 4 bis 20 C-Atomen, einen ω-H-Perfluoralkylrest mit 4 bis 20 C-Atomen, oder einen Rest der Formel $R_f'SO_2NR_3$ oder der Formel $R_f'\ CH_2CH_2SO_2NR_3$, worin $R_f'$ einen Perfluoralkylrest mit 4 bis 20 C-Atomen und $R_3$ = H oder ein $C_{1-4}$-Alkyl ist,
XBr, Cl oder J,
aeine ganze Zahl von 1 bis 4 und
beine Zahl von 0 bis 20,
dadurch gekennzeichnet, daß ein Fluoralkohol-Epihalogenhydrin-Addukt der folgenden Formel 2

$$R_f(CH_2)_aO-(CH_2\underset{CH_2X}{CH}O)_b-CH_2\underset{CH_2X}{CH}OH\qquad (2)\quad ,$$

worin $R_f$, X, a und b die genannten Bedeutungen haben,
zur Abspaltung von Halogenwasserstoff unter Cyclisierung der Halogenhydringruppe des Adduktes mit Alkalimetall- oder Erdalkalimetallhydroxiden in Form einer wäßrigen Lösung, in Gegenwart von Phasentransferkatalysatoren und bei einem pH-Wert von 9 bis 12 umgesetzt und die gebildete, angestrebte Epoxidverbindung aus der Reaktionsmischung isoliert wird.

2. Verfahren nach Anspruch 1, dadurch gekennzeichnet, daß die Umsetzung mit Alkalimetall- oder Erdalkalimetallhydroxid in Form einer 1 bis 40 gew.-%igen wäßrigen Lösung durchgeführt wird.

3. Verfahren nach Anspruch 1 oder 2, dadurch gekennzeichnet, daß die Umsetzung bei einem pH-Wert von 10 bis 11 durchgeführt wird.

4. Verfahren nach einem oder mehreren der Ansprüche 1 bis 3, dadurch gekennzeichnet, daß die Umsetzung bei einer Temperatur von 30 bis 100 °C durchgeführt wird.

5. Verfahren nach Anspruch 1, dadurch gekennzeichnet, daß die Umsetzung in der Weise durchgeführt wird, daß das Fluoralkohol-Epihalogenhydrin-Addukt, dispergiert in Wasser, und der Phasentransferkatalysator in einem Reaktionsgefäß vorgelegt werden, die Mischung auf eine Temperatur von 40 bis 80 °C erhitzt wird und bei dieser Temperatur zur Mischung die zur Abspaltung von Halogenwasserstoff aus der Halogenhydringruppe des Adduktes stöchiometrisch erforderliche Menge an Alkalimetallhydroxid oder Erdalkalimetallhydroxid in Form einer 4 bis 20 gew.-%igen wäßrigen Lösung dazugegeben wird und ein pH-Wert von 10 bis 11 eingehalten wird.

6. Verfahren nach einem oder mehreren der Ansprüche 1 bis 5, dadurch gekennzeichnet, daß ein Fluoralkohol-Epihalogenhydrin-Addukt gemäß Formel 2 eingesetzt wird, wobei $R_f$ ein Perfluoralkylrest mit 6 bis 16 C-Atomen, X Br oder Cl, a 2 und b 0 bis 10 ist.

7. Perfluoralkyl enthaltende Epoxide der folgenden Formel 3

$$R_f(CH_2)_aO-(CH_2\underset{\displaystyle CH_2X}{CH}O)_{b'}-CH_2\underset{\diagdown O \diagup}{CH-CH_2} \qquad (3),$$

worin bedeuten

$R_f$ einen Perfluoralkylrest mit 4 bis 20 C-Atomen, einen ω-H-Perfluoralkylrest mit 4 bis 20 C-Atomen, oder einen Rest der Formel $R'_f\ SO_2NR_3$ oder der Formel $R'_f\ CH_2CH_2SO_2NR_3$, worin $R'_f$ ein Perfluoralkylrest mit 4 bis 20 C-Atomen und $R_3$ = H oder ein $C_{1-4}$-Alkyl ist,

XBr, Cl oder J,

aeine ganze Zahl von 1 bis 4

$b'$ eine Zahl von 1 bis 20.

8. Perfluoralkyl enthaltende Epoxide nach Anspruch 7, wobei $R_f$ ein Perfluoralkylrest mit 6 bis 16 C-Atomen, X Br oder Cl, a 2 und $b'$ 1 bis 10 ist.

## Claims

1. A process for the preparation of perfluoroalkyl-containing epoxides of the following formula 1

$$R_f(CH_2)_aO-(CH_2\underset{\displaystyle CH_2X}{CH}O)_b-CH_2\underset{\diagdown O \diagup}{CH-CH_2} \qquad (1),$$

wherein $R_f$ denotes a perfluoroalkyl radical having 4 to 20 carbon atoms, an ω-H perfluoroalkyl radical having 4 to 20 carbon atoms, or a radical of the formula $R'_fSO_2NR_3$ or the formula $R'_fCH_2CH_2SO_2NR_3$, wherein $R'_f$ is a perfluoroalkyl radical having 4 to 20 carbon atoms and $R_3$ is H or a $C_{1-4}$-alkyl, X denotes Br, Cl, or I, a denotes a whole number from 1 to 4, and b denotes a number from 0 to 20, comprising reacting a fluoroalcohol-epihalohydrin adduct of the following formula 2

$$R_f(CH_2)_aO-(CH_2\underset{\displaystyle CH_2X}{CH}O)_b-CH_2\underset{\displaystyle CH_2X}{CH}OH \qquad (2),$$

wherein $R_f$, X, a, and b have the meanings mentioned, with alkali metal hydroxides or alkaline earth metal hydroxides in the form of an aqueous solution to eliminate the hydrogen halide with cyclization of the halohydrin group of the adduct, in the presence of phasetransfer catalysts and at a pH of 9 to 12 and isolating the desired epoxide compound formed from the reaction mixture.

2. The process as claimed in claim 1, wherein the reaction is carried out with an alkali metal hydroxide or alkaline earth metal hydroxide in the from of a 1 to 40% strength by weight aqueous solution.

3. The process as claimed in claim 1 or 2, wherein the reaction is carried out at a pH of 10 to 11.

4. The process as claimed in one or more of claims 1 to 3, wherein the reaction is carried out at a temperature of 30 to 100°C.

5. The process as claimed in claim 1, wherein the reaction is carried out by introducing the fluoroalcohol-epihalohydrin adduct, dispersed in water, and the phase-transfer catalyst into a reaction vessel, heating the mixture to a temperature of 40 to 80°C and to the mixture adding at this temperature the stoichiometrically necessary amount of alkali metal hydroxide or alkaline earth metal hydroxide, in the form of a 4 to 20% strength by weight aqueous solution, for the elimination of hydrogen halide from the halohydrin group of the adduct and maintaining a pH of 10 to 11.

6. The process as claimed in one or more of claims 1 to 5, wherein a fluoroalcohol-epihalohydrin adduct according to formula 2 is employed, where $R_f$ is a perfluoroalkyl radical having 6 to 16 carbon atoms, X is Br or Cl, a is 2, and b is 0 to 10.

7. A perfluoroalkyl-containing epoxide of the following formula 3

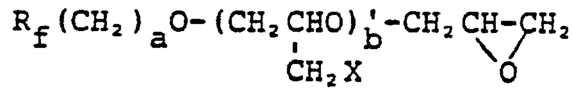 (3) ,

wherein $R_f$ denotes a perfluoroalkyl radical having 4 to 20 carbon atoms, an ω-H perfluoroalkyl radical having 4 to 20 carbon atoms, or a radical of the formula $R'_fSO_2NR_3$ or the formula $R'_fCH_2CH_2SO_2NR_3$, wherein $R'_f$ is a perfluoroalkyl radical having 4 to 20 carbon atoms and $R_3$ is H or a $C_{1-4}$-alkyl, X denotes Br, Cl, or I, a denotes a whole number from 1 to 4, and b' denotes a number from 1 to 20,

8. A perfluoroalkyl-containing epoxide as claimed in claim 7, where $R_f$ is a perfluoroalkyl radical having 6 to 16 carbon atoms, X is Br or Cl, a is 2, and b' is 1 to 10.

**Revendications**

1. Procédé de préparation d'époxydes perfluoralkylés de formule 1 ci-dessous:

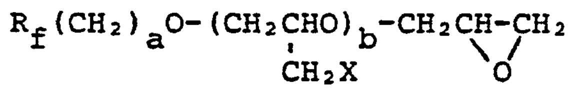 (1) ,

(dans laquelle: $R_f$ désigne un radical perfluoralkyle en $C_4$ à $C_{20}$, un radical ω-H-perfluoralkyle en $C_4$ à $C_{20}$ ou bien un radical $R'_fSO_2NR_3$ ou $R'_fCH_2CH_2SO_2NR_3$, $R'_f$ désignant un radical perfluoralkyle en $C_4$ à $C_{20}$ et $R_3$ l'hydrogène ou un alkyle en $C_1$–$C_4$, X désigne Br, Cl ou I, a un entier de 1 à 4 et b un nombre de 0 à 20), procédé caractérisé en ce que l'on fait réagir un produit d'addition d'un alcool fluoré et d'une épihalogénhydrine, de formule 2:

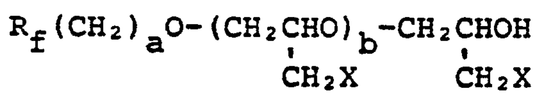 (2) ,

pour en éliminer l'halogénure d'hydrogène, avec cyclisation, du groupe d'halogénhydrine, avec un hydroxyde de métal alcalin ou alcalino-terreux en solution aqueuse en présence d'un catalyseur de transfert de phases, à un pH de 9 à 12, puis on isole le composé époxydique formé du mélange de réaction.

2. Procédé selon la revendication 1, caractérisé en ce que l'on effectue la réaction avec une solution aqueuse de 1 à 40% en poids de l'hydroxyde de métal alcalin ou alcalino-terreux.

3. Procédé selon la revendication 1 ou 2, caractérisé en ce que l'on opère à un pH de 10 à 11.

4. Procédé selon une ou plusieurs des revendications 1 à 3, caractérisé en ce que l'on effectue la réaction entre 30 et 100°C.

5. Procédé selon la revendication 1, caractérisé en ce que pour effectuer la réaction on met dans un récipient de réaction le produit d'addition dispersé dans de l'eau et le catalyseur, on porte le mélange entre 40 et 80°C et pour éliminer l'halogénure d'hydrogène du groupe d'halogénhydrine du produit d'addition on lui ajoute à cette température la proportion stoechiométrique nécessaire de l'hydroxyde de métal alcalin ou alcalino-terreux en solution aqueuse de 4 à 20% en poids, en maintenant un pH de 10 à 11.

6. Procédé selon une ou plusieurs des revendications 1 à 5, caractérisé en ce que l'on opère avec un produit d'addition fluoro-alcool-épihalogénhydrine de formule 2 dont $R_f$ est un perfluoralkyle en $C_6$ à $C_{16}$ X est le brome ou le chlore, a le nombre 2 et b un nombre de 0 à 10.

7. Epoxydes perfluoralkylés de formule 3 ci-dessous:

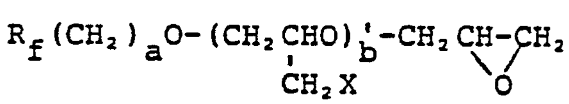 (3) ,

(dans laquelle: $R_f$ désigne un radical perfluoralkyle en $C_4$ à $C_{20}$, un radical en ω-H-perfluoralkyle en $C_4$ à $C_{20}$ ou bien un radical $R'_fSO_2NR_3$ ou $R'_fCH_2CH_2SO_2NR_3$, $R'_f$ désignant un radical perfluoralkyle en $C_4$ à $C_{20}$ et $R_3$ l'hydrogène ou un alkyle en $C_1$–$C_4$, X désigne le brome, le chlore ou d'iode, a est un entier de 1 à 4 et b un nombre de 1 à 20.

8. Epoxydes perfluoralkylés selon la revendication 7 dans lesquels $R_f$ est un radical perfluoralkyle en $C_6$ à $C_{16}$, X le brome ou le chlore, a le nombre 2 et b un nombre de 1 à 10.